# EUROPEAN PATENT APPLICATION

(11) **EP 0 948 940 A1**
(43) Date of publication of application: **13.10.1999**
(21) Application number: 99301789.6
(22) Date of filing: 09.03.1999
(51) Int. Cl.: A61B 19/02, G06M 1/10, A61F 13/44

(54) **Disposal container for detecting, distinguishing and counting objects**

(30) Priority: 07.04.1998 US 56531
(71) Applicant: Szymaitis, Dennis W., Pittsburgh, Pennsylvania 15205 (US)
(72) Inventor: Szymaitis, Dennis W., Pittsburgh, Pennsylvania 15205 (US)
(74) Representative: Marshall, John Grahame

(57) **Abstract**

A container for receiving, detecting, distinguishing and counting marked objects and particularly marked surgical objects which each carry a marker made of a selected nonmagnetostrictive, soft magnetic material which will emit known specific selected high harmonic frequencies when exposed to an alternating electromagnetic field. That emission will cause a change in the alternating electromagnetic field which can be correlated to the presence of only the selected nonmagnetostrictive, soft magnetic material. The container comprises a receptacle (40) and, adjacent an entrance opening (41) to the receptacle, a generator (42) for generating an alternating electrical field and a detector (44) for detecting changes in that alternating field as the marked articles are placed or dropped into the receptacle (40). The container permits an automatic count of surgical instruments, swabs, sponges etc used in an operating theatre without the need to subject the patient to any scanning radiation.

## Description

### Background of the Invention

### 1. Field of the Invention

The invention relates to a container which detects, distinguishes and counts marked objects placed into the container in an operating room using a detection device which responds to an alternating electromagnetic field.

### 2. Description of the Prior Art

Despite precautions, surgeons still occasionally leave surgical objects such as sponges and, less frequently, small surgical tools in their patients after an operation. Areas which are badly injured tend to have a great amount of blood which may cover the surgical objects, making the objects hard to locate. Also, objects may find their way under an organ. This is most likely to occur in surgical areas such as the abdomen which is large and has many organs.

The prior art discloses use of X-ray opaque material positioned on the surgical devices in order that after the surgery is completed and the wound is closed, an X-ray can be taken to insure that no surgical objects were left within the patient. Although this detection method is effective, it is cumbersome. Most operating rooms do not have X-ray machines. Hence, the patient must be taken to another room. There the patient often must be moved from his gurney to an X-ray table for X-rays to be taken. If a surgical object is detected after an X-ray has been taken, the patient must be returned to the operating room. Then, the cavity or incision must be reopened to remove the surgical object and then reclosed. This second surgery can cause a great deal of trauma to the patient, preventing optimum healing. Examples of surgical sponges which are marked by radiopaque material are disclosed in United States Patent No. 2,190,432 to Lewison, United States Patent No. 2,698,270 to Mesek, United States Patent No. 4,185,626 to Jones et al., and United States Patent No. 4,205,680 to Marshall.

Manual counting of the sponges and other surgical objects after the surgery is completed is also used to prevent those objects from being left in body cavities. This is not a foolproof method. Fatigue, poor handwriting, and misreading of numbers will occur during operations lasting 4 to 12 hours when dealing with badly damaged patients. Consequently, miscounts occur as a result of human error.

Surgical objects are counted not only to insure that no such objects are left in the human body, but also to prevent such objects from being inadvertently discarded. There have been instances where a surgical item costing several thousand dollars has been discarded with the surgical trash.

Presently, many operating rooms follow a practice of placing all used and soiled sponges, drains, packs and other objects in a holding container. At the end of the operation the container is emptied and the objects that had been contained therein are counted. Blood on the discarded objects may contain infectious disease viruses. Therefore, each time a soiled surgical object is handled there is a risk of accidental self inoculation. Consequently it is desirable to minimize the handling of such objects, not handle each discarded object twice as in this current practice.

Because of the risks of infection and in an effort to minimize human error, the art has developed several methods of marking objects for automatic detection. Many of those methods rely upon markers which respond to a magnetic or electromagnetic field.

Greenberg in United States Patent No. 3,587,583 attempts to overcome the problems of leaving surgical objects within the human body. He proposes to mark the surgical object with a permanently magnetized material. A surgeon performs an operation in the normal manner. Before closing the incision the surgeon probes for the presence of a surgical object with a magnetic field detector means which generates an electric signal which is modified in the presence of a magnetic field. If the marked object is present, the magnetic field of the magnetic marker is sensed by the magnetic field detector means, which modifies the electric signal. Yet, an operating room has many types of equipment which generate permanent magnetic fields. The presence of those fields can activate the magnetic field detector means, giving false detection. Because of its unreliability in an operating room, Greenberg's device is not a practical solution to the problem.

In United States Patent No. 5,057,095, Fabian proposes to mark surgical instruments with a marker adapted to produce identifying signal characteristics when exposed to an alternating magnetic field. He discloses three types of resonant markers that are able to resonate at a certain preselected frequency. The first marker is a magnetomechanical device comprised of a permanent magnet overlaying a magnetostrictive metal strip in a plastic housing. The magnetostrictive strip vibrates when the marker is exposed to an alternating electromagnetic field, and its resonance is detected when the frequency of the applied field reaches a predetermined value. However, such devices are very sensitive to pressure and stress, which will inhibit them. Since a body cavity is under some pressure and the marker may be stressed during surgery, this type of marker is not reliable for use as a marker for surgical objects. The second proposed type is an electromechanical circuit comprised of an air coil, with or without a ferrite core, and a resonant structure such as a piezoelectric crystal. As the first type, this type of marker can be adversely affected by pressure and stress because its principle of detection relies on a electro-mechanical resonance; therefore, a piezoelectric crystal type marker is also unsatisfactory. The third type of marker proposed by Fabian is an electromagnetic LCR circuit. This type of marker can be either built out of discrete components or made of a flexible printed circuit. In the former case, this unit is expensive to build and bulky, and it is impractical for surgical sponges. In the latter case, due to its high electrical resonance frequency this type of marker can be adversely affected by the presence of metal objects and conductive media. Because the human body is conductive, this type of marker is also impractical for surgical sponges. Consequently, none of the markers proposed by Fabian, nor the Greenberg marker, has been available on the market.

In United States Patent No. 5,045,071, McCormick teaches about the use of magnetic materials for accurately locating the position of a catheter which has been inserted into a blood vessel. At column 9, lines 12-16, the patent cross references U.S. Patents 4,416,289, 4,431,005 and 4,445,501 for an explanation of the general method of detection. At column 5, lines 41-52, the '005 patent explains that a distortion of the magnetic field indicates the presence of the catheter. Thus, the McCormick patent teaches that merely a change in the magnetic field is a sufficient indicator of the position of the marked object. However, McCormick's measurements can be affected by the presence of other nearby magnetic and conductive materials. Hence, McCormick's technique can and likely will provide "false positives" as to the presence or the position of the marked object.

In my United States Patent No. 5,456,718 I disclose a marker for surgical objects made of a selected nonmagnetostrictive, soft magnetic material which will emit known specific selected harmonic frequencies when exposed to an alternating magnetic field. I further taught that a variety of devices could be used to detect the presence of a magnetized market attached to an surgical object left in a patient. However, I did not teach nor then realize that a container could be provided for used surgical objects which could detect, distinguish and count the objects as they are placed in the container. Consequently, it would not be necessary to routinely subject patients to a detection device as they left the operating room. Only when a count indicating that an object was missing would there be a need to scan the patient.

Thus, there is a container for receiving, detecting distinguishing and counting surgical objects as they are retired from use an operating theater where the objects have been marked with a material that can be readily identified before the patient leaves the operating suite. The detector in the container must not give false positives or otherwise be ineffective in the presence of magnetic and electromagnetic fields of the type commonly produced in the operating room. Such a container and method should also be useful outside the operating room in environments where objects must be counted or detected and in which magnetic or electromagnetic fields are present.

### 3. Techniques for Detecting Electromagnetic Material

There are different ways of providing and detecting what we can call generically an "electromagnetic marker." The cited prior art references all use materials which respond to an electromagnetic field. In order for a material to respond to an electromagnetic field and therefore to create "detectable changes" of the electromagnetic field, a material has to have at least one of the physical properties of electrical conductivity, moderate to high magnetic permeability, and magnetostriction (in general associated with moderate magnetic permeability). Moderate magnetic permeability is defined as a permeability comprised of between 5,000 and 20,000 and high magnetic permeability as a permeability above 20,000. In each case, the response to the electromagnetic field and, therefore, the creation of "detectable changes" of the electromagnetic field are heavily dependent upon the geometry and size of the marker. In addition, the response to the electromagnetic field depends upon the intensity and frequency of the electromagnetic field.

In general, a magnetic material subject to an electromagnetic field of known and fixed frequency fₒ responds to the applied electromagnetic field by creating "changes" of the intensity of the applied field and by creating harmonics of the frequency fₒ. If the material is electrically conductive, it responds by creating not only "changes" of the intensity of the applied field but also "changes" of the phase of the field. In addition, if the material is magnetostrictive, the electromagnetic field creates strains or stress in the material, and the material responds to it by creating a frequency-dependent "change" of the intensity and of the phase of the applied field. Therefore, there are three methods of detection. First, one can simply look for a change of intensity and/or phase in an applied magnetic field, a method which can only be used for detection of the position of an object at a distance comparable to the size of the object. McCormick uses this method. Second, one could look for the frequency of the applied field to reach a predetermined value that is the electromechnical resonance frequency of the marker. Fabian discloses a magnetomechanical device which uses this technique. Finally, one could look for particular harmonics generated by a material in the presence of an applied magnetic field. This method has never been used in a medical environment. Indeed, the teaching of Heltemes in United States Patent No. 4,857,891 indicates that the art has generally failed to recognize that "open-strip" markers made of selected nonmagnetostrictive materials which generate specific harmonic frequencies upon application of a unidirectional electromagnetic field can be used to identify the presence of particular articles.

Heltemes discloses a Magnetic Marker for Electronic Article Surveillance Systems having multiple filaments randomly dispersed in a sheet-like substrate so as to be substantially parallel to the plane thereof. "The filaments are selected of low coercive force, high permeability material, and the random orientation results in certain filaments intersecting with them being magnetically coupled to other filaments to thereby collect and concentrate lines of flux associated with an applied field of an EAS system into filaments parallel to the field."

To take advantage of a high magnetic permeability material a marker has to be elongated (fiber, long strip, with an aspect ratio length/square root or cross-sectional area of a least 200). Heltemes complies only partially to this requirement, but randomly distributes the fibers. In this respect Heltemes defeats this purpose because the applied electromagnetic field has to be parallel to the magnetic fibers to generate a high enough level of high harmonics to be recognized as marker specific. Moreover, Heltemes' marker is not very well suited for generating high harmonics. Consequently, Heltemes, like others in the prior art, failed to recognize that markers could be created for detection of surgical objects which generate specific, detectable, selected harmonic frequencies.

### Summary of the Invention

I provide a container for receiving and detecting objects which have an attached marker. The container has a generator that produces an alternating electromagnetic field. The marker responds to the presence of that alternating electromagnetic field. That response is detectable as discrete pulses of radio frequencies. The marker is comprised of at least one elongated member made of nonmagnetostrictive, soft magnetic material encapsulated in biocompatible material. Preferably, the magnetic material is an amorphous metal. However, crystalline materials can be used if the encapsulation material has a high flexibility and plasticity.

Detection is made using a pulse detection technique similar to that used in magnetic resonance imaging (MRI), a common and safe diagnostic procedure. As an object is placed into the container, the object is exposed to an alternating electromagnetic field of about 3-4 Oe (oersteds) for a specific time. This field will cause the elongated member to become magnetized and consequently to generate harmonics of the frequency of the applied alternating electromagnetic field. A detector placed nearby will detect the reflected wave form from the marker as sharp signal peaks at the specific frequency of the applied alternating electromagnetic field.

My marker is particularly useful for surgical sponges. However, other surgical tools (e.g., forceps, scalpel), plastic or rubber/polymer surgical implement or equipment used during surgery (e.g., plastic drain, suction tubes), and implants (e.g., artificial veins, artificial arteries, knee replacement) may also be marked.

When the object is a surgical sponge, the marker can be woven into the sponge. When the surgical object is a surgical tool such as forceps, the marker is placed on the tool by an adhesive means.

A microprocessor and memory can be connected to the detector. As objects are detected they are counted. The total is then recorded and can be displayed. A keypad can be provided to allow a user to enter the number of objects that should be placed into the container. A program would then sound an alarm if too few objects were received.

Other details, objects and advantages of the invention will become apparent as the following description of a present preferred embodiment thereof and a present preferred method of practising the same proceeds.

### Brief Description of the Drawing

In the accompanying drawing I have shown a present preferred embodiment of the invention in which

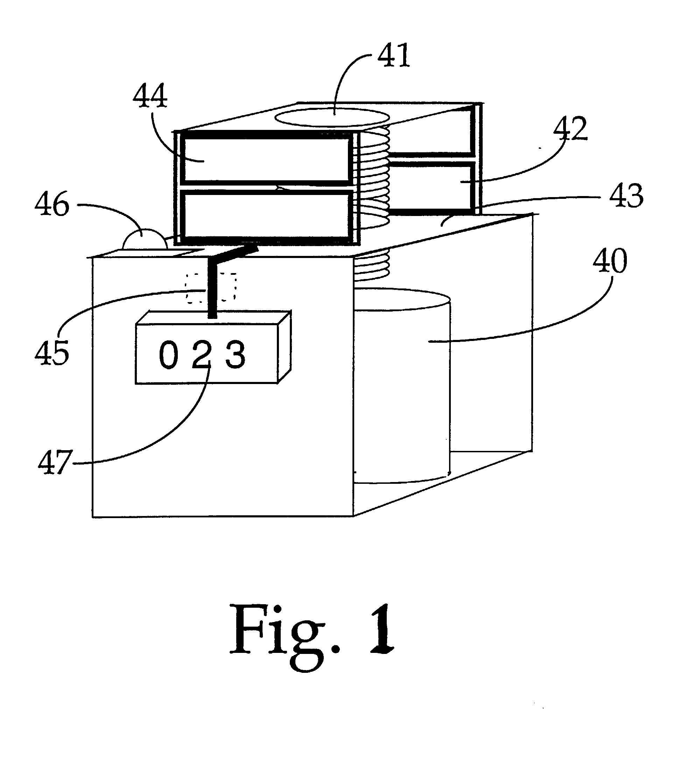

Figure 1 is a perspective view of a container having mounted thereon a detector with associated components for detecting, distinguishing and counting marked objects as they are placed into the container.

The container of this invention is for use with objects having a marker attached thereto, the marker comprised of a material which will emit at least one known specific intensity of harmonic frequencies when exposed to an alternating electromagnetic field thereby causing a change in the alternating electromagnetic field which change can be correlated to the presence of the material. Examples of such objects are illustrated and fully described in my European Patent Specification EP-A-747016.

Marked objects can be detected and counted at the time of disposal using a waste container according to the invention as shown in Figure 1. A detector 42 is placed on the top 43 of a waste container 40 near opening 41. Another detector 44 is placed on the container 40, opposite the first detector 42. At least one of the detectors includes an electromagnetic field generator. Whenever a marked object is dropped into the container 40 the marker on the object will emit a signal that is detected by the detector. The detector will then emit a corresponding signal to a control system to indicate that a marked object has been discarded. Preferably, the control system is attached to or within the container as indicated by chain line box 45. The control system causes a display 47 to show the number of marked items that have been discarded. Although not shown, the control system could be connected to an external computer which utilizes or stores the number of discarded, marked items. This information could be used for patient billing or other purposes. The container 40 may have a warning light 46 or LED display which is illuminated whenever an object having a particular marker is detected. If surgical sponges which are to be discarded have a marker that evokes one response and other objects are marked with a marker that prompts a different response, the control system can be designed to illuminate the light only when marked objects which are not to be discarded pass the detector.

The detectors 42 and 44 that are placed on the container 40, could also be used in other locations, such as at a doorway or disposal chute, to detect and count the passage of marked objects.

In Figure 1 I have shown certain preferred orientations and geometries of the elements that generate the electromagnetic field and detect the harmonics produced by the marker. Those skilled in the art will recognize that there are many other possible embodiments. For example, a frame inductor antenna can be placed opposite a search coil. Two frame inductor antennas could be placed at right angles to two search coil detectors. The electromagnetic field could be created by two frame inductor antennas placed near a search coil detector. Other types of coils could be used, either for generating the electromagnetic field or for detecting the signal from a marker.

## Claims

1. A container for receiving and detecting objects having a marker attached thereto, the marker comprised of a material which will emit at least one known specific intensity of harmonic frequencies when exposed to an alternating electromagnetic field thereby causing a change in the alternating electromagnetic field which change can be correlated to the presence of the material, the container comprising:
a) a receptacle (40) having a chamber sized to receive marked objects and an opening (41) through which the marked objects can be placed into the container;
b) a generator (42) attached to the receptacle (40) for generating an alternating electromagnetic field which field will change in the presence of the marker; and
c) a detector (44) attached to the receptacle (40) for detecting a change in the alternating electromagnetic field which change can be correlated to the presence of the material of the marker thereby indicating that at least one object has been detected.

2. A container according to claim 1 for receiving and detecting objects having a marker attached thereto, the marker comprised of at least one elongated body of a selected nonmagnetostrictive soft magnetic material, such that said marker will emit a spectrum of harmonics which includes detectable high order harmonics when exposed to an alternating electromagnetic field thereby causing a change in the alternating electromagnetic field which change can be correlated to the presence of the selected nonmagnetostrictive, soft magnetic material, wherein the detector (44) is adapted to detect a change in the alternating electromagnetic field which change can be correlated to the presence of the selected nonmagnetostrictive, soft magnetic material thereby indicating that at least one object has been detected.

3. A container according to claim 1, for receiving and detecting objects having a marker attached thereto, the marker comprised of at least two mutually parallel elongated bodies of a selected nonmagnetostrictive, soft magnetic material, such that said marker will emit a spectrum of harmonics which includes detectable high order harmonics, and which will emit at least one known specific intensity of harmonic frequencies when exposed to an alternating electromagnetic field thereby causing a change in the alternating electromagnetic field which change can be correlated to the presence of only the selected nonmagnetostrictive, soft magnetic material, wherein the detector (44) is adapted to detect a change in the alternating electromagnetic field which change can be correlated to the presence of only the selected nonmagnetostrictive, soft magnetic material thereby indicating that at least one object has been detected.

4. A container according to any of claims 1 to 3, wherein the generator (42) and the detector (44) are attached adjacent the opening (41) of the receptacle (40).

5. A container according to any of claims 1 to 4, also comprising a control system (45) connected to the detector (44).

6. A container according to claim 5, also comprising a display (47) connected to the control system (45).

7. A container according to claim 5, also comprising a warning light (46) connected to the control system (45).

8. A container according to any of claims 5 to 7, wherein the control system (45) contains a program for counting marked objects as those objects are placed into the receptacle.

9. A container according to claim 8, wherein the programs further compares the number of counted marked objects with an expected number of marked objects.

10. A container according to any preceding claim, for use with a range of marked objects in which at least some emit a different specific harmonic frequency than other marked objects, wherein the detector (44) is capable of distinguishing different harmonic frequencies.
